(19) Europäisches Patentamt / European Patent Office / Office européen des brevets

(11) **EP 2 581 103 A1**

(12) **DEMANDE DE BREVET EUROPEEN**

(43) Date de publication:
**17.04.2013 Bulletin 2013/16**

(51) Int Cl.:
***A61M 16/12*** *(2006.01)* ***A62B 27/00*** *(2006.01)*

(21) Numéro de dépôt: **12184951.7**

(22) Date de dépôt: **19.09.2012**

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**
Etats d'extension désignés:
**BA ME**

(30) Priorité: **12.10.2011 FR 1159211**

(71) Demandeur: **Air Liquide Medical Systems**
**92160 Antony (FR)**

(72) Inventeur: **Boulanger, Thierry**
**37000 Tours (FR)**

(74) Mandataire: **Pittis, Olivier**
**L'Air Liquide, S.A.,**
**Direction de la Propriété Intellectuelle,**
**75, Quai d'Orsay**
**75321 Paris Cedex 07 (FR)**

(54) **Procédé de calibration d'un appareil de distribution de gaz alimenté par une source de NO**

(57) L'invention porte sur un procédé de calibration d'un appareil de distribution de gaz comprenant un dispositif analyseur de gaz (6) comprenant au moins un premier élément de mesure (615) de concentration en NO, ledit appareil étant connecté fluidiquement à une source de NO contenant un mélange gazeux comprenant une proportion source donnée (Psource) de NO gazeux, et par ailleurs à une source d'air exempt de NO, dans lequel on procède selon les étapes d'établir la valeur zéro du premier élément de mesure (615), et d'établir ou d'ajuster une valeur de concentration de référence (Pref) en NO du premier élément de mesure (615) à partir du NO issu de la source de NO et d'air ambiant.

FIG.1

EP 2 581 103 A1

**Description**

La présente invention concerne un procédé de calibration ou d'étalonnage d'un appareil de distribution de gaz comprenant un dispositif analyseur de gaz comprenant des éléments de mesure de concentration en NO et en $NO_2$, en particuliers des capteurs de NO et $NO_2$.

L'oxyde nitrique ou monoxyde d'azote (ci après « NO ») est un gaz incolore qui, lorsqu'il est inhalé, dilate les vaisseaux sanguins des poumons et augmente l'oxygénation du sang en favorisant les échanges gazeux. Cette propriété est exploitée pour traiter l'hypertension artérielle pulmonaire.

A cette fin, le NO est ajouté aux gaz inspirés par le patient souffrant d'hypertension pulmonaire selon une posologie déterminée, fixée par le médecin, qui varie généralement entre 1 et 100 ppm en volume, notamment en fonction du patient considéré.

Le gaz inhalé par le patient contenant du NO est fourni par un dispositif de délivrance, tel qu'un ventilateur mécanique, comme décrit par exemple par US-A-5,558,083.

Un tel appareil, qui est alimenté par une ou des bouteilles contenant un mélange d'azote ($N_2$) et de NO à une concentration typiquement de l'ordre de 200 à 800 ppm en volume de NO, comprend généralement un module d'injection de NO directement placé dans la branche inspiratoire d'un circuit patient connecté à ses extrémités respectivement au ventilateur mécanique et à un patient.

Le module d'injection inclut un capteur de débit qui mesure le débit délivré par le ventilateur mécanique et retourne cette mesure à l'appareil d'administration de NO pour que celui-ci en déduise un débit de NO à délivrer, en relation avec la posologie désirée.

Le débit ainsi déterminé est assuré au moyen d'une électrovanne proportionnelle associée à un capteur de débit interne à l'appareil et est administré au moyen d'une ligne d'injection située dans le dit module d'injection, en aval du système de mesure. Le mode d'administration du NO est préférentiellement continu en ce sens que l'appareil recueille de manière continue la mesure de débit du gaz circulant dans la branche inspiratoire du circuit patient et actualise en permanence le débit de NO à délivrer afin de respecter la posologie désirée.

Toutefois, dans certaines conditions, l'appareil de délivrance peut opérer une administration sous forme de débit pulsé, discontinu, par exemple en cas de faibles débits issus du ventilateur mécanique.

A des fins sécuritaires, un appareil de délivrance de NO dispose généralement d'une ligne de prélèvement de gaz permettant de prélever une portion du gaz inspiré par le patient, typiquement 100 ml/min environ, et préférentiellement raccordée au niveau de la pièce Y du circuit patient, afin de l'analyser et de déterminer les teneurs en gaz dans le circuit patient, notamment :

- la concentration en NO qui doit être la plus proche possible de la posologie désirée,
- la concentration en oxygène ($O_2$) qui est variable et dépend des réglages effectués d'une part sur le ventilateur mécanique (enrichissement en $O_2$) et de la posologie désirée. En effet, une forte posologie associée à un faible enrichissement en oxygène peut en effet se traduire par un mélange dit hypoxique pouvant entraîner des dommages importants au patient.
- la concentration en dioxyde d'azote ($NO_2$) qui doit être la plus faible possible et détectée à de très faibles concentrations, typiquement de 0 à 5 ppm en volume. En effet, le $NO_2$, qui résulte de la réaction entre le NO et l'$O_2$ est un gaz extrêmement toxique pouvant entraîner de graves lésions chez le patient, y compris à faible concentration de l'ordre de quelques ppm en volume.

Le document EP-A-872254 décrit un analyseur utilisable pour mesurer les teneurs en les différents gaz susmentionnés, c'est-à-dire NO, $NO_2$ et $O_2$, comportant une pompe qui vient prélever du gaz à analyser en pièce Y du circuit patient. La pompe, qui est située en aval des éléments ou capteurs de mesure, permet de faire mettre le gaz au contact des capteurs pour réaliser la mesure. Le gaz utilisé pour les mesures est ensuite évacué vers l'atmosphère ambiante.

Dans cet appareil, les éléments de mesure de concentration mettent en oeuvre une technologie de type électrochimique qui n'est pas exempte de contrainte puisque ce type de capteur souffre d'une dérive et nécessite une calibration périodique.

En fait, la calibration ou l'étalonnage à « zéro » des capteurs consiste à présenter aux éléments de mesure un mélange gazeux de référence dépourvu de NO et de $NO_2$ afin de déterminer leur réponse nominale au repos, c'est-à-dire sans excitation découlant de la présence de ces gaz.

Ceci est généralement réalisé en prélevant l'air ambiant où les teneurs de NO et $NO_2$ sont faibles car fortement diluées. Pour ce faire, le dispositif met en oeuvre une électrovanne qui, en fonctionnement normal, assure la liaison entre le point de prélèvement du gaz et la pompe mais qui, dans le cadre d'un étalonnage de zéro des capteurs, est commutée pour assurer une liaison entre l'air ambiant et la pompe qui fournit alors aux capteurs un mélange gazeux sans NO, ni $NO_2$.

Par ailleurs, le gain des capteurs qui consiste à présenter aux éléments de mesure un mélange gazeux calibré de NO et $NO_2$ afin de déterminer leur réponse à une concentration en NO ou $NO_2$ donnée.

Ceci se fait à intervalles de temps réguliers, par exemple tous les mois ; bien entendu, la fréquence de calibration peut être supérieure ou inférieure à un mois, notamment en fonction de la fréquence d'utilisation de l'appareil.

Or, une procédure de calibration peut être assez longue, c'est-à-dire de l'ordre de 20 minutes, voire davantage, et nécessite des moyens conséquents, en particulier des bouteilles étalonnées à une concentration de gaz donnée, typiquement une bouteille de NO dosé à 40 ppmv et une bouteille de $NO_2$ dosé à 10 ppmv.

La phase de calibration du capteur de NO consiste alors à connecter la bouteille de NO à 40 ppmv à l'entrée de l'analyseur, à régler un débit donné de NO au moyen d'un débitmètre et à injecter ce débit pendant un temps suffisant pour obtenir un signal stable aux bornes du capteur. La technologie électrochimique étant relativement lente, plusieurs dizaines de secondes peuvent être nécessaires.

On procède de la même façon avec la bouteille de $NO_2$ et le capteur associé.

On comprend aisément que la fréquence préconisée de réalisation de ces phases de calibration ainsi que le temps consacré à leur réalisation imposent au personnel hospitalier une charge de travail non négligeable et immobilise régulièrement les appareils.

En outre, l'emploi de bouteilles calibrées de NO et de $NO_2$ représente un coût d'exploitation non négligeable pour l'hôpital.

Enfin, chacune des bouteilles-étalons dispose d'une date de péremption au delà de laquelle la concentration indiquée sur la bouteille pourrait ne pas être respectée, entraînant par là une erreur d'étalonnage. Il convient donc à chaque fois, pour des raisons de sécurité, de s'assurer que les mélanges gazeux étalons sont toujours utilisables ou, à défaut, les remplacer, ce qui a aussi un coût non négligeable.

Partant de là, le problème qui se pose est de remédier à cette problématique de calibration périodique en proposant un appareil de délivrance de gaz, en particulier de mélanges $NO/N_2$, qui intègre des éléments de mesure de concentration des gaz, c'est-à-dire un dispositif analyseur de gaz, permettant d'espacer la fréquence de calibration périodique et surtout de simplifier cette procédure, lequel ne nécessite pas l'usage de bouteille-étalon de gaz de calibration, en particulier de bouteilles étalonnées contenant du NO ou du $NO_2$ en teneurs précises.

La solution de la présente invention porte alors sur un procédé de calibration, encore appelé procédé d'étalonnage, d'un appareil de distribution de gaz comprenant un dispositif analyseur de gaz comprenant au moins un premier élément de mesure de concentration en NO, ledit appareil étant connecté fluidiquement à une source de NO contenant un mélange gazeux comprenant une proportion source donnée (Psource) de NO gazeux, et par ailleurs à une source d'air exempt de NO, procédé dans lequel on procède selon les étapes de :

A) établir la valeur zéro du premier élément de mesure en procédant selon les sous-étapes successives de :

i) mettre le premier élément de mesure en contact avec de l'air exempt de NO,
ii) opérer au moins une mesure de concentration en NO au moyen du premier élément de mesure,
iii) recueillir au moins un signal de mesure de concentration en NO,
iv) affecter audit signal de mesure de concentration en NO recueilli en iii), une valeur de concentration égale à 0, et

B) établir ou ajuster une valeur de concentration de référence (Pref) en NO du premier élément de mesure en procédant selon les sous-étapes successives de :

i) mettre au moins le premier élément de mesure en contact avec le mélange gazeux contenant une concentration de référence (Pref) donnée de NO gazeux issu de la source de NO, avec $Pref = \alpha.Psource$, où $\alpha$ est un facteur de dilution préfixé compris entre 1 et 1/50,
ii) opérer au moins une mesure de concentration en NO du mélange gazeux contenant une concentration de référence (Pref) donnée de NO, au moyen dudit premier élément de mesure,
iii) recueillir au moins un signal de mesure de concentration de référence (Pref en NO,
iv) affecter audit signal de mesure de concentration de référence en NO obtenu en B) iii), ladite valeur de concentration de référence (Pref) en NO.

**[0024]** Selon le cas, le procédé de calibration ou d'étalonnage selon la présente invention peut comprendre l'une ou plusieurs des caractéristiques techniques suivantes :

- le premier élément de mesure de concentration en NO est un capteur de NO, par exemple de type PID AH de Alphasense Ltd.
- le deuxième élément de mesure de concentration en $NO_2$ est un capteur de $NO_2$, par exemple de type P/N 706 de Synkera Technologies Ltd.

- la source d'air exempt de NO et/ou de $NO_2$ est de l'air ambiant, c'est-à-dire de l'air prélevé à l'atmosphère ambiante. En effet, l'air ambiant est en général exempt ou quasi-exempt de NO et $NO_2$, c'est-à-dire qu'il n'en contient pas ou alors en des proportions négligeables, c'est-à-dire typiquement compris entre 0,01 et 0,1 ppm.
- le facteur de dilution préfixé $\alpha$ est compris entre 1 et 1/50, de préférence entre 1 et 1/10. Ainsi, lorsque $\alpha=1$, alors Pref = Psource, ce qui signifie que le mélange gazeux NO issu de la source n'est pas dilué avec de l'air après sa sortie de la source de NO (le NO est par contre généralement dilué dans de l'azote au sein de la source). A l'inverse, si $\alpha=1/10$, alors Pref = 0,1 x Psource, ce qui signifie que le mélange gazeux NO issu de la source est dilué au 1/10e avec de l'air, après sa sortie de la source de NO.
- la dilution du NO avec de l'air est obtenue en ajustant le ou les débits de NO et d'air, de préférence l'ajustage de débit d'air est opéré via une pompe commandée par les moyens de pilotage du module.
- le mélange gazeux NO issu de la source contient toujours une proportion de NO (avant dilution) d'au moins 50 ppm en volume, le reste étant typiquement de l'azote.
- à l'étape A) ii), la mesure de la teneur en NO permettant d'établir la valeur « 0 » (zéro) du premier élément de mesure, est opérée après une durée D donnée suffisante pour purger la ligne de gaz à laquelle est raccordé le premier élément de mesure de tout NO résiduel. Ceci permet d'éviter de fausser la mesure dudit premier élément de mesure.
- à l'étape B) iv), le fait d'affecter au signal de mesure de concentration de référence en NO une valeur de concentration de référence (Pref) en NO permet de caler ou recaler le premier élément de mesure.
- les signaux de mesure et les valeurs de concentrations associées auxdits signaux de mesure obtenus aux étapes A) iv) et B) iv) sont mémorisés dans des moyens de mémorisation, par exemple

une mémoire RAM ou analogue, en particulier une mémoire faisant partie des moyens de pilotage de l'installation, tel une Unité Central de Traitement ou CPU (en abréviation anglaise).

- durant la sous-étape B) iii) :

    a) on traite le signal de mesure de concentration de référence (Pref) en NO en opérant une comparaison dudit signal de mesure de concentration de référence (Pref) en NO, corrigé dudit facteur de dilution $\alpha$, avec une ou plusieurs valeurs préenregistrées représentatives de plusieurs concentrations sources (Psource) de NO données,
    b) en fonction du résultat de la comparaison opérée en sous-étape a), on détermine la concentration réelle en NO de la source de NO, et
    c) on affecte à ladite concentration réelle de NO de la source la valeur de concentration de référence (Pref) en NO corrigée dudit facteur de dilution $\alpha$.

- durant la sous-étape B) iii), la ou les valeurs préenregistrées de NO représentatives de plusieurs concentrations sources (Psource) de NO données sont comprises entre 100 et 1000 ppm en volume de NO, typiquement la ou les valeurs de NO préenregistrées sont égales à 225, 450 et/ou 800 ppm en volume de NO (le reste étant considéré comme étant de l'azote).
- il comporte en outre une étape C) supplémentaire telle que :

    C) établir la valeur zéro du deuxième élément de mesure (620) en procédant selon les sous-étapes successives de :

        i) mettre le deuxième élément de mesure en contact avec de l'air ambiant exempt de $NO_2$,
        ii) opérer au moins une mesure de concentration en $NO_2$ au moyen du deuxième élément de mesure,
        iii) recueillir au moins un signal de mesure de concentration en $NO_2$,
        iv) affecter audit signal de mesure de concentration en $NO_2$ recueilli en iii), une valeur de concentration égale à 0.

- les étapes A) et C) sont simultanées ou quasi-simultanées et opérées avec de l'air exempt de NO et de $NO_2$, de préférence de l'air ambiant.
- il comporte en outre une étape supplémentaire D) suivante :

    D) établir une valeur de concentration en NO prédéfinie (Pdet) par le biais du premier élément de mesure en procédant selon les sous-étapes successives de :

        i) mélanger du NO gazeux issu de la source de NO avec une proportion donnée ajustable d'air de manière à obtenir un mélange gazeux de NO dilué avec de l'air contenant une proportion intermédiaire (Pint) connue de NO avec :

$$Pref > Pint > 0$$

ii) mettre au moins le premier élément de mesure en contact avec le mélange gazeux de NO dilué avec de l'air,
iii) opérer au moins une mesure de concentration intermédiaire en NO au moyen dudit premier élément de mesure,
iv) recueillir au moins un signal de mesure de concentration intermédiaire en NO,
v) répéter les étapes D) iii) et iv) en ajustant la source d'air de manière à recueillir sur le premier élément de mesure un signal de mesure de concentration en NO égal à une valeur prédéfinie Pdet, avec Pref $\geq$ Pdet > 0.

- la source de NO contient un mélange gazeux comprenant une proportion source donnée (Psource) de NO gazeux mélangé avec un gaz inerte, en particulier de l'azote.
- il comporte en outre une étape supplémentaire E) suivante :

E) établir une valeur de concentration en $NO_2$ ($P_{NO2}$) du deuxième élément de mesure (620) en procédant selon les sous-étapes successives de :

i) stopper toute injection préalable de mélange gazeux NO/air contenant du NO et de l'air de manière à obtenir, dans le dispositif analyseur de gaz, une concentration homogène (Pdet) en NO et négligeable en $NO_2$,
ii) attendre une durée T donnée suffisante pour qu'au moins une partie du NO présent dans le dispositif analyseur de gaz, s'oxyde et forme du $NO_2$ au contact de l'oxygène contenu dans l'air en mélange avec le NO,
iii) après ladite durée T, mettre au moins le deuxième élément de mesure en contact avec le mélange gazeux contenant du $NO_2$ s'étant formé pendant la durée T donnée,
iv) opérer au moins une mesure de concentration en $NO_2$ au moyen dudit deuxième élément de mesure,
v) recueillir au moins un signal de mesure de concentration en $NO_2$,
vi) affecter audit signal de mesure de concentration en $NO_2$, une valeur de concentration en $NO_2$ donnée déterminée à partir d'une ou plusieurs valeurs de référence de concentration en $NO_2$ préétablies correspondant à des concentrations en $NO_2$ formé pendant la durée T donnée dans le mélange NO/air.

- les signaux de mesure de concentration et/ou les mesures de concentration qui leur sont affectées sont mémorisés, de préférence au sein de moyens de mémorisation par exemple une unité de type CPU.
- à l'étape D), les valeurs de référence de concentration en $NO_2$ préétablies correspondant à des concentrations en $NO_2$ formé pendant la durée T donnée comprennent un ou des tableaux de correspondance préétablis et mémorisés.
- avantageusement, on corrige les valeurs de pression mesurées en prenant en compte la pression atmosphérique et/ou la température régnant dans le module.
- les signaux mesurés par les premier et deuxième éléments de mesure sont des signaux de tension électrique.
- les signaux mesurés par les premier et deuxième éléments de mesure sont traités par les moyens de pilotage de l'appareil de distribution de gaz, en particulier par un ou des microprocesseurs.
- le mélange du NO gazeux issu de la source de NO avec de l'air ambiant de manière à obtenir un mélange gazeux de NO dilué avec de l'air est réalisé au moyen d'une pompe, de préférence une pompe à membrane par exemple de type V200 de Xavitech AB.
- la pompe servant à réaliser au moins un mélange NO/air est pilotée par les moyens de pilotage de l'appareil de distribution de gaz, lesdits moyens de pilotage agissant sur la pompe en réponse à un ou plusieurs signaux transmis par l'un ou plusieurs des premier et deuxième capteurs.
- la proportion source donnée (Psource) de NO gazeux est comprise entre 100 et 1000 ppm en volume, de préférence le reste du mélange gazeux est de l'azote.

Le procédé de calibration selon la présente invention peut être mis en oeuvre pour étalonner un appareil de distribution de NO muni de capteurs à NO et à $NO_2$, voire à $O_2$, utilisable dans la mise en oeuvre d'une méthode de traitement thérapeutique de l'adulte ou de l'enfant, notamment des nouveau-nés, souffrant d'hypertension artérielle pulmonaire que l'on traite par administration par voie inhalée d'oxyde nitrique ou monoxyde d'azote permettant de dilater les vaisseaux sanguins des poumons et augmenter l'oxygénation du sang en favorisant les échanges gazeux pulmonaires chez ces patients.

L'invention va maintenant être décrite plus en détail en rapport avec la Figure annexée qui est une représentation schématique d'un mode de réalisation d'un appareil de distribution de NO apte à mettre en oeuvre le procédé de calibration selon la présente invention.

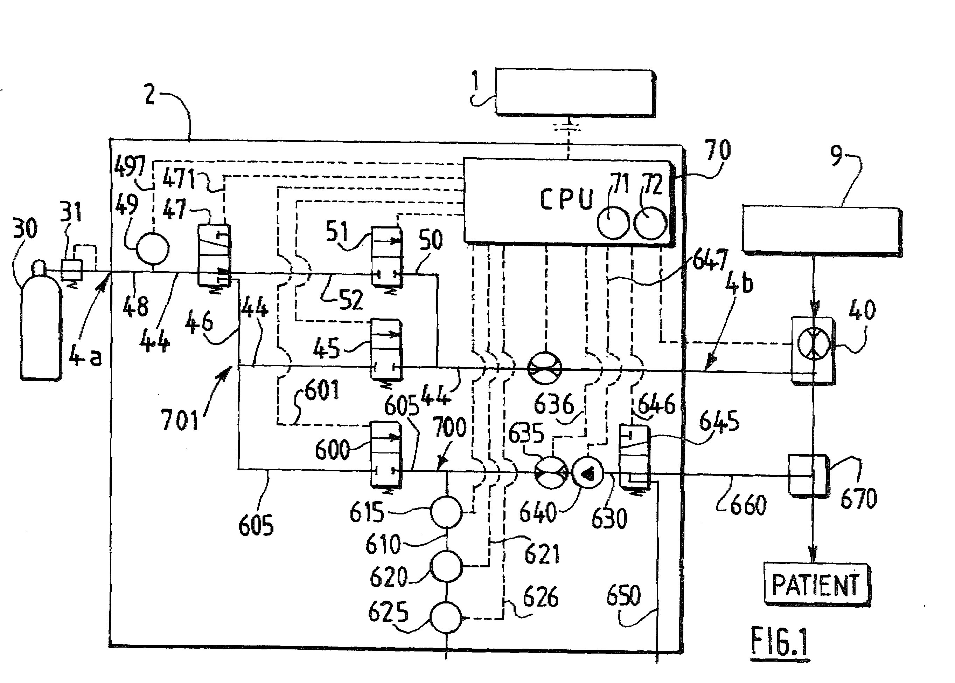

Plus précisément, la Figure 1 représente un schéma d'un mode de réalisation d'une installation de distribution de NO à un patient incluant un appareil 2 de distribution de NO comprenant des capteurs de NO 615 et $NO_2$ 620 qui sont calibrés ou étalonnés grâce au procédé de calibration automatique selon la présente invention.

Plus précisément, cette installation de distribution de NO comprenant un ventilateur 9 pour délivrer un gaz respiratoire contenant de l'oxygène, typiquement de l'air ou un mélange $O_2/N_2$ contenant au moins 21% en volume d'$O_2$, au sein d'un circuit patient 630 reliant le ventilateur 9 à un patient, et comprenant par ailleurs un appareil 2 de distribution de NO connecté fluidiquement audit circuit patient 630 via une ligne d'injection de gaz 44 et une ligne de prélèvement 660.

Une source 30 de NO gazeux, telle une bouteille de gaz, alimente la ligne d'injection 44 de gaz de l'appareil 2 avec un gaz contenant du NO, typiquement un mélange NO/azote contenant un mélange gazeux formé d'azote et de 1 à 1000 ppmv de NO.

**[0031]** La ligne d'injection 44 de NO et ligne de prélèvement 660 du gaz inhalé sont pneumatiquement indépendantes l'une de l'autre, c'est-à-dire non reliées directement l'une à l'autre.

En fonctionnement normal, l'appareil 2 enrichit avec du NO gazeux (i.e. un mélange NO/azote), le mélange gazeux respiratoire, par exemple de l'air ou un mélange $O_2/N_2$, provenant du ventilateur 9 selon une posologie déterminée, typiquement par un médecin ou similaire.

La posologie désirée, habituellement comprise entre 10 et 80 ppm en volume de NO, est réglée sur l'appareil 2 au moyen d'organes de réglage, tels que des boutons de réglages ou bien une interface homme/machine comprenant un écran tactile par exemple.

Afin de délivrer la posologie souhaitée, le dispositif 2 est alimenté par la (ou des) bouteille 30 contenant une concentration donnée de NO, variant généralement entre 100 et 800 ppmv, le reste étant un gaz inerte, tel de l'azote. Par exemple, des mélanges NO/$N_2$ à 225 ou à 450 ppmv sont disponibles auprès de la société Air Liquide Santé.

Pour assurer la posologie souhaitée, l'appareil 2 met en oeuvre une ligne d'injection 44 qui assure le transport et la délivrance de la quantité appropriée de NO dans le circuit patient 630.

Par ailleurs, est également prévu une ligne de secours 50 qui est utilisée lors d'une défaillance majeure du dispositif 2 et vise à ne pas interrompre brutalement la thérapie par NO inhalé, en cas de défaillance de la ligne d'injection 44, car une telle interruption pourrait avoir des conséquences cliniques importantes pour le patient.

En outre, l'appareil comprend un analyseur de gaz 6 qui servent à déterminer les concentrations en NO, $NO_2$ et $O_2$ à partir d'échantillons de gaz prélevés dans le circuit patient 630, via la ligne d'analyse 660 qui alimente l'analyseur 6 en lesdits échantillons gaz. Ceci permet de s'assurer qu'au cours du traitement, la posologie de NO est bien respectée et que par ailleurs, les teneurs en $O_2$ et $NO_2$ correspondent à des valeurs désirées.

L'analyseur 6 comprend des éléments de mesure 615, 620, 625, c'est-à-dire des capteurs, servant à déterminer les teneurs en NO, $NO_2$ et $O_2$ des échantillons prélevés dans le circuit 630, au niveau d'un site de prélèvement 670.

La calibration ou étalonnage des éléments de mesure 615, 620, 625 doit être faite à intervalles réguliers, en général tous les mois. Pour ce faire, on procède selon le procédé de la présente invention qui sera détaillé ci-après. Les gaz ayant servi à la calibration sont ensuite envoyés à l'atmosphère via une ligne de mise à l'atmosphère ambiante 610, c'est-à-dire un tronçon de passage de gaz en communication fluidique avec la ligne de prélèvement 660, sur laquelle sont agencés les prises de mesure des éléments de mesure 615, 620, 625 de l'analyseur 6.

L'appareil 2 de distribution de NO/$N_2$ comprend en outre une liaison pneumatique spécifique, appelée ligne de calibration 605, aménagée entre les lignes d'injection 44 de NO et de prélèvement 660 du gaz inhalé, et permettant de réaliser les calibrations des éléments de mesure 615, 620, 625, c'est-à-dire des capteurs, de l'analyseur de gaz 6.

La ligne de calibration 605 est raccordée fluidiquement (en 701) à la ligne d'injection de gaz 44 et (en 700) à la ligne d'analyse de gaz 660 de manière à permettre un passage direct de gaz de la ligne d'injection de gaz 44 à la ligne d'analyse de gaz 660, portant le tronçon 610 sur lequel se trouvent les éléments de mesure et d'analyse 615, 620, 625.

L'agencement de cette ligne de calibration 605 permet donc d'assurer une communication pneumatique, c'est-à-dire le passage direct de gaz à base de NO, entre les lignes d'injection 44, de secours 50 et l'analyseur de gaz 6.

La circulation de gaz au sein de la ligne de prélèvement 660 est contrôlée par une électrovanne 645 commandée par un dispositif de pilotage 70, telle une unité à microprocesseur (ou CPU), qui participe notamment à la réalisation des étapes de calibration automatique des différents éléments de mesure 615, 620, 625 de l'analyseur 6 de gaz.

Le dispositif de pilotage 70 permet également de déterminer la concentration en NO du système d'alimentation en gaz du dispositif; et d'opérer périodiquement une purge du système d'alimentation en gaz via la ligne de mise à l'atmosphère 610 ambiante qui est connectée fluidiquement à la ligne 660.

Par ailleurs, un capteur de pression 49 est agencé sur le tronçon amont 48 de la ligne d'injection 44 situé au niveau de l'entrée 4a de gaz du dispositif 2, afin de mesurer la pression gazeuse fournie par le système d'alimentation en gaz, comprenant la bouteille 30 et un détendeur 31 situé en sortie de bouteille 30, typiquement une pression de l'ordre de 3,5 bar. La valeur de pression déterminée par le capteur 49 est retournée au dispositif de pilotage 70 comprenant une ou plusieurs cartes de commande, par le biais d'une liaison électrique 491, ce qui permet de s'assurer que le dispositif 2 est correctement alimenté en gaz contenant du NO.

**[0046]** En condition normale de fonctionnement, c'est-à-dire sans recours à la ligne de secours 50, une électrovanne à 3 voies 47 est pilotée grâce à la liaison 471 par le dispositif de pilotage 70 de telle manière à garantir une liaison entre le tronçon d'admission des gaz 48 et une première électrovanne 45, appelée électrovanne de dosage, située sur le tronçon de transmission 46 de la ligne d'injection 44, entre l'électrovanne à 3-voies 47 et la sortie 4b de la ligne d'injection 44.

L'électrovanne à 3 voies 47 permet de mettre en relation le tronçon d'admission des gaz 48 et l'électrovanne de calibration 600 par l'intermédiaire du tronçon de transmission 46 de la ligne d'injection 44 et la ligne de calibration 605 qui est raccordée fluidiquement à la ligne d'injection de gaz 44 et à la ligne d'analyse de gaz 610, via le tronçon 605, pour permettre un passage direct de gaz contenant du NO de la ligne d'injection de gaz 4 à la ligne d'analyse de gaz 660, en particulier au tronçon 610, comprenant le dispositif d'analyse 6.

L'électrovanne de dosage 45 est alimentée en gaz provenant de la source de NO 30 et assure de manière classique, le dosage des gaz dans la ligne d'injection 44 en fonction de la posologie réglée par l'utilisateur 1, du débit gazeux issu du ventilateur mécanique 9 et mesuré par le module d'injection 40 et de la concentration en NO de la bouteille 30 du système d'alimentation en gaz.

En condition normale de fonctionnement, l'électrovanne 3 voies 47 est pilotée par le dispositif de pilotage 70 de manière à ce que l'électrovanne de secours 51 ne soit pas alimentée. Seule une condition de défaillance majeure du dispositif, telle que la perte de toute commande électrique, déclenche le système de secours de sorte qu'un débit de NO traverse l'électrovanne de secours 51 afin de continuer à administrer au patient une quantité donnée de NO.

**[0050]** Par ailleurs, lorsque le dispositif 2 administre une posologie donnée de NO au patient, l'électrovanne 600 de calibration est commandée en position fermée par la carte de commande du dispositif de pilotage 70 via la liaison électrique 601 de sorte que la ligne de calibration 605 ne soit pas alimentée par le mélange gazeux NO/N$_2$ provenant du système 30 d'alimentation en gaz.

Une quatrième électrovanne 645, appelée électrovanne de zéro, agencée sur la ligne de prélèvement 660 entre l'entrée de prélèvement 6a et l'électrovanne 600 de calibration, est également commandée par la carte de commande du dispositif de pilotage 70, via une liaison électrique 646, de telle manière que la communication fluidique au sein de la ligne de prélèvement 660 soit permise et assurée jusqu'à l'analyseur de gaz 6.

La quatrième électrovanne 645 est en fait une électrovanne à 3 voies ayant une voie 650 reliée à l'atmosphère et ses deux autres voies reliées à la ligne de prélèvement 660.

La voie 650 reliée à l'atmosphère sert à prélever de l'air atmosphérique qui est utilisé lors des étapes de calibration, comme expliqué ci-après, pour établir les valeurs zéro des capteurs de NO 615 et de NO$_2$ 620, et aussi utiliser pour éventuellement diluer la concentration en NO lors desdites étapes de calibration.

Par ailleurs, la ligne de prélèvement 660 est connectée au plus proche du patient par le biais d'un raccord 670, par exemple une pièce en Y du circuit patient 630, afin que les gaz analysés soient représentatifs de ce qui est inhalé par le patient.

La ligne de prélèvement de gaz 660 comporte en outre une pompe 640 aspirante et un capteur de débit 635 agencés entre la quatrième électrovanne 645 et l'électrovanne 600 de calibration.

Le prélèvement de gaz est rendu possible grâce à la pompe 640 qui est commandée par la carte de commande du dispositif de pilotage 70 via une liaison électrique 641 et qui aspire le gaz sous un débit donné, et est contrôlé au moyen du capteur de débit 635 coopérant avec la carte de commande du dispositif de pilotage 70 pour récupérer, via la liaison 636, la (les) valeur de débit au sein de la ligne 660. Il s'agit dès lors d'un système régulé puisque la carte de commande du dispositif de pilotage 70 pilote la pompe 640 afin que le débit prélevé et mesuré par le capteur de débit 635 reste constant. On notera que le capteur de débit 635 peut être directement intégré dans la pompe et renvoyé par celle-ci via la liaison 641 à la carte de commande du dispositif de pilotage 70 comme un élément de mesure

Le débit ainsi prélevé, typiquement entre 50 et 200 ml/min, est ensuite analysé par un ensemble d'éléments de mesure 615, 620, 625 de l'analyseur 6, avant d'être évacué de l'appareil et rejeté, par exemple à l'atmosphère à travers la ligne de mise à l'atmosphère 610, encore appelée canal d'analyse.

Cette analyse du gaz prélevé dans le circuit patient 630 permet de fournir des informations sur les concentrations en gaz envoyées vers le patient, c'est-à-dire les concentrations en NO, NO$_2$ et O$_2$. Ces informations sont par exemple affichées sur un écran et/ou mémorisées.

La pompe 630 sert également à prélever de l'air atmosphérique, via le tronçon 650, qui est utilisé lors des étapes de calibration comme expliqué ci-après.

Comme déjà mentionné, l'analyseur de gaz 6 comporte plusieurs capteurs ou éléments de mesure 615, 620 et 625 permettant de mesurer la teneur en NO, NO$_2$ et O$_2$ dans le flux gazeux prélevé.

Le premier élément de mesure 615 permet de mesurer spécifiquement les concentrations en NO et de les transmettre sous forme de signal électrique via la liaison électrique 616 au dispositif de pilotage 70 du dispositif 2. Il s'agit par exemple d'un capteur de type électrochimique mais on peut lui préférer une technologie basée sur la photo-ionisation. Par exemple, on peut utiliser le capteur PID AH disponible auprès de Alphasense, qui permet de mesurer une large plage de concentration en gaz, typiquement 0 à 700 ppm en volume de NO avec une résolution de l'ordre de 0,1 ppmv

Le second élément de mesure 620 permet de mesurer les concentrations en NO$_2$ du gaz prélevé et ensuite d'envoyer à la carte de commande du dispositif 70, les signaux de concentrations en NO$_2$ mesurées, via une liaison électrique 621. Le second élément de mesure 620 est par exemple un de type CMOS tel que le P/N 706 de Synkera Technologies, qui permet de mesurer une plage de l'ordre de 0 à 10 ppmv de NO$_2$ avec une résolution de l'ordre de 0.1 ppmv.

Outre les concentrations en NO et NO$_2$ il est également nécessaire de mesurer celle en O$_2$. Ceci est réalisé par le biais d'un troisième élément de mesure 625, par exemple le capteur d'oxygène commercialisé par S4MS sous la référence SMSI 02 Sensor, qui envoie la concentration en O$_2$ à la carte de commande du dispositif 70 via la liaison 626.

Toutes les concentrations mesurées et transmises au dispositif de pilotage 70 peuvent être affichées sur un écran et/ou mémorisées.

Or, comme déjà mentionné, une utilisation prolongée et/ou fréquente de l'appareil peut entraîner une dérive desdits éléments de mesure ou capteurs 615, 620, 625 dans l'appréciation des concentrations en gaz, à savoir en NO, NO$_2$ et O$_2$.

Il est dès lors impératif de procéder à un étalonnage ou calibrage desdits capteurs 615, 620, 625. La procédure de calibration et d'étalonnage de l'analyseur 6 se fait de la manière suivante par mise en oeuvre du procédé de l'invention.

On observe qu'en dehors de la procédure de calibration, l'électrovanne 600 de calibration est commandée en position fermée par la carte de commande 70 via la liaison électrique 601 de manière à ce que le canal de calibration 605 ne soit pas alimenté par le mélange NO/N$_2$ contenu dans le système d'alimentation en gaz. De la même manière, l'électrovanne de zéro 645 est commandée par la carte de commande 70 via la liaison 646 de manière à ce que la communication entre le canal de prélèvement 660 et le canal de pompe 630 soit réalisée.

Les éléments de mesure 615 et 620 renvoient chacun à la carte de commande 70 (via les liaisons 616 et 621) une information U différente, généralement une tension. La carte de commande opère alors une transformation fonction de cette information U afin d'en déduire la concentration en gaz, ici le NO et le NO$_2$.

Deux paramètres sont alors à considérer pour réaliser cette transformation : l'offset U$_{NO}$ et U$_{NO2}$ des capteurs, c'est à dire la tension des capteurs en l'absence de tout stimulus (par exemple la présence de NO ou de NO$_2$) et leur gain G$_{NO}$ et G$_{NO2}$. Ceci se traduit par les formules (1) ci-après.

$$[1] \qquad \begin{aligned} N_{NO} &= G_{NO} \cdot f(U - U_{NO}) \\ N_{NO_2} &= G_{NO2} \cdot g(U - U_{NO2}) \end{aligned}$$

Or ces deux paramètres, offset et gain, sont susceptibles d'évoluer dans le temps et nécessitent alors d'être recalculés.

L'objet de la présente invention est ainsi de décrire un procédé de calibration mettant en oeuvre une séquence de différentes phases s'appuyant sur l'architecture pneumatique susmentionnée et permettant une calibration automatique des deux éléments de mesure 615 et 620, c'est-à-dire des capteurs de NO et NO$_2$,..

La première phase consiste ainsi à déterminer les offsets U$_{NO}$ et U$_{NO2}$ de capteurs qui auraient dérivé. Pour ce faire, on commute l'électrovanne 3-voies de zéro 645 par le biais de la carte de commande 70, via la liaison électrique 646, de manière à ce que celle-ci garantisse une liaison entre le canal d'atmosphère 650 et le canal de pompe 630.

La carte de commande 70 pilote alors la pompe 640 via la liaison 641 afin de prélever un débit représentatif de l'air ambiant, exempt de tout NO et NO$_2$, et à la concentration en O$_2$ de l'air, c'est à dire 21 % en volume.

Le débit prélevé pourra être compris entre 0 et 400 ml/min, de préférence de l'ordre de 100 ml/min, en ajustant la commande de la pompe 640 de manière à ce que le capteur de débit 635 retourne à la carte de commande 70 via la liaison 636 un signal approprié.

Après avoir observé un temps suffisant pour débarrasser le canal d'analyse 610 de toute concentration résiduelle en NO ou NO$_2$, le débit gazeux formé d'air atmosphérique traversant les éléments de mesure 615 et 620 permet de réaliser le zéro de ces derniers puisqu'il est dépourvu de NO et de NO$_2$.

La carte de commande 70 recueille alors via les liaisons 616 et 621, des signaux respectivement U'$_{NO}$ et U'$_{NO2}$ de telle sorte à les réinjecter dans l'équation [1] et on obtient alors l'équation (2) suivante.

$$[2] \quad \begin{aligned} N_{NO} &= G_{NO} \cdot f(U - U'_{NO}) \\ N_{NO_2} &= G_{NO2} \cdot g(U - U'_{NO2}) \end{aligned}$$

Cette première phase achevée, il reste à déterminer le nouveau gain des capteurs 615 et 620.

A cet effet, la carte de commande 70 désactive via la liaison 621, l'élément de mesure 620, c'est à dire ne l'alimentera plus. En effet, l'élément 620 requiert pour détecter finement les concentrations en $NO_2$ d'être porté à haute température, par exemple 250°C. Une telle température peut être de nature à affecter la séquence suivante, aussi la pompe 640 continuera d'injecter son débit dans le canal d'admission 610 pendant un temps suffisant pour refroidir l'élément 620, par exemple 30 sec. A ce terme, la phase suivante peut débuter.

Cette phase suivante est le coeur du procédé de la présente invention car elle repose sur les oxydes d'azote, à savoir NO et $NO_2$ qui sont des composés faisant l'objet de transformations oxydatives, en présence d'oxygène.

Ainsi, il est connu que le NO a de fortes affinités pour l'oxygène et ils forment ensemble du $NO_2$ selon la réaction (3) suivante :

$$[3] \quad NO + \frac{1}{2}O2 \underset{K'}{\overset{K}{\rightleftarrows}} NO2$$

Cette réaction (3) est dépendante des conditions ambiantes de pression et de température à travers une constante de dissociation K. On note que les températures basses favorisent la formation de $NO_2$ alors que les températures élevées la ralentissent.

La réaction inverse de transformation du $NO_2$ en NO existe également et est aussi dépendante des conditions ambiantes de pression et de température à travers une constante de dissociation K'. On note, à l'inverse, que les températures hautes favorisent la formation de NO, alors que les températures basses la ralentissent.

Ainsi, à des températures basses, par exemple 25°C, la constante K est bien supérieure à K', ce qui engage une formation de $NO_2$ au détriment du NO alors qu'à température élevée, par exemple 600°C l'inverse se produira.

Il apparaît à travers ces mécanismes de transformation que la concentration en O2 présente dans le gaz influence également la vitesse de réaction, ce qui peut être mis en équation sous la forme (4) suivante :

$$[4] \quad \begin{aligned} y_{NO}(t) &= \frac{\sqrt{K'}(1+e^{at})}{(\sqrt{K'}-\sqrt{K \cdot P_{O_2}}+(\sqrt{K'}+\sqrt{K \cdot P_{O_2}})e^{at})} \\ y_{NO_2}(t) &= 1 - y_{NO}(t) \end{aligned}$$

Avec :

$$a = 2X_0\sqrt{K \cdot K' \cdot P_{O_2}}$$

$$K(T) = \alpha e^{(\frac{\lambda}{T})}$$

$$K'(T) = \alpha' e^{(\frac{\lambda'}{T})}$$

Où:

- t est le temps, exprimé en s.
- K et K' s'expriment en s$^{-1}$

Les coefficients $\alpha$, $\alpha'$ ainsi que $\lambda$ et $\lambda'$ sont déterminés expérimentalement selon le positionnement des différents éléments constituant l'analyseur de gaz 6. Par exemple $\alpha$ = 0,3 ; $\alpha'$ = 3.10$^7$ ; $\lambda$ = 1200 et $\lambda'$ = -13000.

- $X_0$ est la fraction molaire initiale en NO, c'est-à-dire la concentration en ppm divisée par 1000000.
- $P_{O_2}$ est la pression partielle en bar en $O_2$, c'est-à-dire la concentration en oxygène multipliée par la pression ambiante.

Selon ces équations, pour une concentration initiale en NO de 50 ppmv et une pression partielle d'oxygène de 0.21 bar à 298K (pression partielle de l'oxygène dans l'air sous une pression de 1 bar), les mécanismes de transformation aboutissent à une formation de 5 ppmv de $NO_2$ en 470 sec, c'est-à-dire environ 8 minutes.

Ceci est pleinement mis à profit dans le cadre du procédé de calibration du capteur de $NO_2$ 620 selon la présente invention, comme expliqué ci-après.

On se reporte alors à la Figure qui illustre l'architecture pneumatique utilisée pour comprendre comment s'effectue la calibration des éléments de mesure 615 et 620 selon le procédé de l'invention.

Dans l'ordonnancement de cette séquence de calibration, l'électrovanne 3 voies d'admission 47 garantit une liaison entre l'admission des gaz 48 et l'électrovanne de calibration 600, il est possible de piloter cette électrovanne de calibration 600 par le biais de la carte de commande 70 via la liaison électrique 601 afin de la commuter en position ouverte de sorte que le NO issu de la source de gaz 30, circule dans le canal d'analyse 610.

L'électrovanne de calibration 600 aura été soigneusement sélectionnée de telle manière à ce qu'en position ouverte et sous une pression d'alimentation de 4 bars, mesurée par le capteur de pression 49, le débit circulant dans le canal d'analyse 610 soit de compris entre 10 et 100 ml/min et préférentiellement de l'ordre de 25 ml/min.

Si un débit de 25 ml/min circulant dans le canal d'analyse 610 contient le NO sous une concentration donnée, une dilution est possible par le biais de la pompe 640.

A cet effet, l'électrovanne 3 voies de zéro 645 reste commutée par le biais de la carte de commande 70 via la liaison électrique 646 de telle manière à ce que celle-ci garantisse une liaison entre le canal d'atmosphère 650 et le canal de pompe 630.

Le débit d'air ambiant prélevé alors par la pompe est exempt ou quasi totalement exempt de tout NO et $NO_2$ et est par ailleurs à la concentration en $O_2$ de l'air, c'est-à-dire 21% en volume.

La carte de commande 70, qui alimente la pompe 640 via la liaison électrique 641 reçoit également via 636 l'information de débit issue du capteur 635. Cette disposition permet alors de moduler la commande de la pompe afin que son débit $Q_{pompe}$ soit tel que la concentration en NO dans le mélange constitué des débits issus de l'électrovanne de calibration 600 et de la pompe 640 soit égal à un dixième de la concentration en NO de la source 30, selon la formule (5) suivante :

$$[5] \qquad N_{cal} = N_b / 10 \Rightarrow Q_{pompe} = 9 \cdot Q_{cal}$$

Avec

- $Q_{cal}$ le débit en mL/min délivré par l'électrovanne de calibration, par exemple 25mL/min
- $N_B$ la concentration en NO de la bouteille d'alimentation 30
- $N_{cal}$ la concentration en NO résultant de la dilution par 10 de la concentration en NO de la bouteille

Après avoir observé un temps suffisant pour que la concentration Ncal s'établisse dans l'analyseur de gaz 6, une mesure est réalisée par l'élément 615 et transmise à la carte de commande 70 via la liaison électrique 616 pour y subir la transformation selon l'équation (6) :

$$[6] \qquad N_{NO} = G_{NO} \cdot f(U - U'_{NO})$$

La carte de commande 70, qui dispose maintenant d'une information de concentration $N_{NO}$ en ppm, procède alors au calcul du nouveau gain de l'élément 615.

En effet, la concentration en NO $N_B$ des bouteilles d'alimentation 30 est une valeur discrète, par exemple 225, 450 ou 800 ppm, c'est-à-dire qu'en procédant à une dilution à un dixième de la concentration de la source d'alimentation 30, les concentrations résultantes seront elles-mêmes discrètes et respectivement égales à 22,5, 45 et 80 ppm.

Or, la valeur $N_{NO}$ de concentration oscillera autour d'une de ces valeurs discrètes selon la concentration en NO de la bouteille d'alimentation 30 et l'amplitude de la dérive de l'élément de mesure 615. Ces valeurs discrètes étant préenregistrées, la carte de commande 70 procèdera alors à l'identification de la valeur $N_{CPU}$ la plus proche de la valeur $N_{NO}$ et déterminera alors le nouveau gain de l'élément de mesure 615 (7) :

$$[7] \qquad G'_{NO} = G_{NO} \cdot \frac{N_{CPU}}{N_{NO}}$$

Les paramètres $U'_{NO}$ et $G'_{NO}$ étant définis, le capteur 615 est maintenant parfaitement calibré.

Il est dès lors possible de poursuivre la séquence afin de déterminer le seul paramètre manquant : le nouveau gain de l'élément de mesure 620. Alors que la valeur retournée par l'élément de mesure 615 est maintenant considérée comme fiable, il est possible d'ajuster la commande de la pompe 640 de telle manière à ce que le capteur 615 renvoie via 616 à la carte de commande 70 une concentration désirée en NO que l'on pourra arbitrairement fixer à 50 ppm.

Le choix de porter la concentration en NO à 50 ppm est motivé par l'absence de réponse du capteur 620 pour une telle concentration et par le fait que la teneur résiduelle en $NO_2$ serait alors négligeable.

Plus généralement, dans le cas de l'utilisation d'une bouteille d'alimentation dont la concentration en NO est inférieure à 500 ppm, on peut faire le choix de viser comme mélange une concentration en NO égale à 10% de la concentration de la bouteille. En considérant ainsi, une bouteille d'alimentation 30 sous 200 ppmv, le mélange constitué des débits issus de l'électrovanne de calibration 600 et de la pompe 640 est égal à 20 ppm.

A ce stade de la calibration en $NO_2$ du capteur 620, un mélange à, par exemple, 50 ppm de NO circule dans le canal d'analyse 610. On veille à ce que cette phase dure suffisamment longtemps afin de garantir la purge dudit canal de tout élément étranger, par exemple de NO ou $NO_2$ résiduels.

Or, ce mélange comporte de l'oxygène à hauteur de 21 % en concentration, c'est-à-dire que les éléments principaux de la réaction, NO et $O_2$ sont présents et qu'une réaction de formation de $NO_2$ peut avoir lieu.

Il convient alors d'évaluer les différents paramètres K, K', $X_0$ et $P_{O2}$ susmentionnés, ce qui suppose de disposer d'éléments de mesure tel que la température et pression ambiantes. A cette fin, la carte de commande 70 dispose de deux capteurs 71 et 72, directement placés sur celle-ci et qui fournissent ces deux indications, de température pour 71 et de pression pour 72.

Ainsi, les valeurs de K et K' sont déterminées par la lecture de la température, convertie en degré Kelvin et $P_{O2}$ par la lecture de la concentration en oxygène mesurée par le capteur 625 et renvoyé à la carte de commande 70 via 626 multipliée de la pression ambiante mesuré par 72 et convertie en bar.

Ces informations nourrissent alors les équations [4] ci-avant et l'on peut alors prédire le temps T1 nécessaire pour que s'opère une formation de $NO_2$ jusqu'à atteindre une concentration cible comprise entre 1 et 10 ppm, par exemple de l'ordre de 5 ppm.

Dans cette optique, la carte de commande vient alors couper l'injection de NO en fermant l'électrovanne de calibration 600 via la liaison électrique 601 tandis qu'elle opère simultanément à l'arrêt de la pompe 640. Le volume de gaz piégé dans le canal d'analyse 610 est ainsi laissé en suspension.

Au bout du temps T1 nécessaire à la formation de 5ppm de $NO_2$, la carte de commande 70 réactive l'élément de mesure 620. Après quelques secondes de temps de chauffe il est possible de recueillir via 621 le signal électrique correspondant à une concentration de 5 ppm.

Cette tension, associée à l'offset de l'élément 620 réalisé pendant la phase de zéro permet de déterminer le nouveau gain $G'_{NO2}$ du capteur. La concentration en $NO_2$ sera alors donnée par les nouveaux coefficients de l'équation (8) ci-après:

$$[8] \qquad N_{NO_2} = G'_{NO2} \cdot g(U - U'_{NO2})$$

Bien qu'il existe une relative incertitude quant à la reproductibilité des calibrations, la réaction de conversion oxydative de NO en $NO_2$ est lente, à savoir de l'ordre de 0,1 ppm par dizaine de secondes, ce qui limite l'ampleur de l'imprécision.

Ainsi, dans la plage de temps T1 comprise entre 7 et 9 min pour une température de 298 K et une pression partielle d'$O_2$ de 0.21, l'erreur relative sur la concentration de 5 ppm n'excédera pas 10%, soit ainsi une erreur de l'ordre de 5% à 2,5 ppm, qui est une teneur qu'il est nécessaire de ne pas dépasser pour éviter de graves complications médicales.

Afin de terminer la procédure de calibration, la carte de commande 70 réactive via 641 la pompe 640 afin que celle-ci rince par de l'air le canal d'analyse 610 du $NO_2$ résiduel.

La phase de calibration de l'élément de mesure 620 est alors arrivée à son terme et le dispositif dispose alors d'un capteur reconfiguré et prêt à fournir des informations précises de la teneur en $NO_2$ des gaz patient.

Il apparaît dans la séquence décrite ici que qu'une technologie autre de capteur, par exemple électrochimique pourrait être utilisée dès lors que sa sensibilité croisée au NO ne se manifeste pas pour une concentration de 50 ppm. Dans ce cas, les phases de désactivation puis de réactivation de l'alimentation de l'élément de mesure par la carte de commande 70 ne serait pas nécessaire car une telle technologie électrochimique ne requiert pas d'être portée à température élevée.

## Revendications

1. Procédé de calibration d'un appareil de distribution de gaz comprenant un dispositif analyseur de gaz (6) comprenant au moins un premier élément de mesure (615) de concentration en NO, ledit appareil étant connecté fluidiquement à une source de NO contenant un mélange gazeux comprenant une proportion source donnée (Psource) de NO gazeux, et par ailleurs à une source d'air exempt de NO, procédé dans lequel on procède selon les étapes de :

   A) établir la valeur zéro du premier élément de mesure (615) en procédant selon les sous-étapes successives de :

      i) mettre le premier élément de mesure (615) en contact avec de l'air exempt de NO,
      ii) opérer au moins une mesure de concentration en NO au moyen du premier élément de mesure (615),
      iii) recueillir au moins un signal de mesure de concentration en NO,
      iv) affecter audit signal de mesure de concentration en NO recueilli en iii), une valeur de concentration égale à 0, et

   B) établir ou ajuster une valeur de concentration de référence (Pref) en NO du premier élément de mesure (615) en procédant selon les sous-étapes successives de :

      i) mettre au moins le premier élément de mesure (615) en contact avec le mélange gazeux contenant une concentration de référence (Pref) donnée de NO gazeux issu de la source de NO, avec Pref = $\alpha$ . Psource, où $\alpha$ est un facteur de dilution préfixé compris entre 1 et 1/50,
      ii) opérer au moins une mesure de concentration en NO du mélange gazeux contenant une concentration de référence (Pref) donnée de NO, au moyen dudit premier élément de mesure (615),
      iii) recueillir au moins un signal de mesure de concentration de référence (Pref) en NO, et
      iv) affecter audit signal de mesure de concentration de référence en NO obtenu en B) iii), ladite valeur de concentration de référence (Pref) en NO.

2. Procédé selon la revendication 1, **caractérisé en ce que** durant la sous-étape B) iii) :

   a) on traite le signal de mesure de concentration de référence (Pref) en NO en opérant une comparaison dudit signal de mesure de concentration de référence (Pref) en NO, corrigé dudit facteur de dilution $\alpha$, avec une ou plusieurs valeurs préenregistrées représentatives de plusieurs concentrations sources (Psource) de NO données,
   b) en fonction du résultat de la comparaison opérée en sous-étape a), on détermine la concentration réelle en NO de la source de NO, et
   c) on affecte à ladite concentration réelle de NO de la source la valeur de concentration de référence (Pref) en NO corrigée dudit facteur de dilution $\alpha$

3. Procédé selon la revendication 1, **caractérisé en ce que** le dispositif analyseur de gaz (6) comprend un deuxième élément de mesure (620) de concentration en $NO_2$, et **en ce qu'**il comporte en outre une étape C) supplémentaire telle que :

   C) établir la valeur zéro du deuxième élément de mesure (620) en procédant selon les sous-étapes successives

EP 2 581 103 A1

de :

i) mettre le deuxième élément de mesure (620) en contact avec de l'air ambiant exempt de $NO_2$,
ii) opérer au moins une mesure de concentration en $NO_2$ au moyen du deuxième élément de mesure (620),
iii) recueillir au moins un signal de mesure de concentration en $NO_2$,
iv) affecter audit signal de mesure de concentration en $NO_2$ recueilli en iii), une valeur de concentration égale à 0.

**4.** Procédé selon l'une des revendications précédentes, **caractérisé en ce que** les étapes A) et C) sont simultanées ou quasi-simultanées et opérées avec de l'air exempt de NO et de $NO_2$, de préférence de l'air ambiant.

**5.** Procédé selon l'une des revendications précédentes, **caractérisé en ce qu'il** comporte en outre une étape supplémentaire D) suivante :

D) établir une valeur de concentration en NO prédéfinie (Pdet) par le biais du premier élément de mesure (615) en procédant selon les sous-étapes successives de :

i) mélanger du NO gazeux issu de la source de NO avec une proportion donnée ajustable d'air de manière à obtenir un mélange gazeux de NO dilué avec de l'air contenant une proportion intermédiaire (Pint) connue de NO avec Pref $\geq$ Pint > 0,
ii) mettre au moins le premier élément de mesure (615) en contact avec le mélange gazeux de NO dilué avec de l'air,
iii) opérer au moins une mesure de concentration intermédiaire en NO au moyen dudit premier élément de mesure (615),
iv) recueillir au moins un signal de mesure de concentration intermédiaire en NO,
v) répéter les étapes D) iii) et iv) en ajustant la source d'air de manière à recueillir sur le premier élément de mesure (615) un signal de mesure de concentration en NO égal à une valeur prédéfinie Pdet, avec Pref $\geq$ Pdet > 0.

**6.** Procédé selon l'une des revendications précédentes, **caractérisé en ce que** la source de NO contient un mélange gazeux comprenant une proportion source donnée (Psource) de NO gazeux mélangé avec un gaz inerte, en particulier de l'azote.

**7.** Procédé selon l'une des revendications précédentes, **caractérisé en ce que** la source d'air exempt de NO et/ou de $NO_2$ est l'atmosphère ambiante.

**8.** Procédé selon l'une des revendications précédentes, **caractérisé en ce qu'il** comporte en outre une étape supplémentaire E) suivante :

E) établir une valeur de concentration en $NO_2$ ($P_{NO2}$) du deuxième élément de mesure (620) en procédant selon les sous-étapes successives de :

i) stopper toute injection préalable de mélange gazeux NO/air contenant du NO et de l'air de sorte de manière à obtenir, dans le dispositif analyseur de gaz (6), une concentration homogène (Pdet) en NO et négligeable en $NO_2$,
ii) attendre une durée T donnée suffisante pour qu'au moins une partie du NO présent dans le dispositif analyseur de gaz (6), s'oxyde et forme du $NO_2$ au contact de l'oxygène contenu dans l'air en mélange avec le NO,
iii) après ladite durée T, mettre au moins le deuxième élément de mesure (620) en contact avec le mélange gazeux contenant du $NO_2$ s'étant formé pendant la durée T donnée,
iv) opérer au moins une mesure de concentration en $NO_2$ au moyen dudit deuxième élément de mesure (620),
v) recueillir au moins un signal de mesure de concentration en $NO_2$,
vi) affecter audit signal de mesure de concentration en $NO_2$, une valeur de concentration en $NO_2$ donnée déterminée à partir d'une ou plusieurs valeurs de référence de concentration en $NO_2$ préétablies correspondant à des concentrations en $NO_2$ formé pendant la durée T donnée dans le mélange NO/air.

**9.** Procédé selon l'une des revendications précédentes, **caractérisé en ce que** les signaux de mesure de concentration et/ou les mesures de concentration qui leur sont affectées sont mémorisés, de préférence au sein de moyens de mémorisation par exemple le CPU.

10. Procédé selon l'une des revendications précédentes, **caractérisé en ce qu'à** l'étape D), les valeurs de référence de concentration en $NO_2$ préétablies correspondant à des concentrations en $NO_2$ formé pendant la durée T donnée comprennent un ou des tableaux de correspondance préétablis et mémorisés.

11. Procédé selon l'une des revendications précédentes, **caractérisé en ce que** le mélange du NO gazeux issu de la source de NO avec de l'air ambiant de manière à obtenir un mélange gazeux de NO dilué avec de l'air est réalisé au moyen d'une pompe (640).

12. Procédé selon l'une des revendications précédentes, **caractérisé en ce que** la pompe (640) servant à réaliser au moins un mélange NO/air est pilotée par les moyens de pilotage (70) de l'appareil de distribution de gaz, lesdits moyens de pilotage (70) agissant sur la pompe (640) en réponse à un ou plusieurs signaux transmis par l'un ou plusieurs des premier et deuxième capteurs (615,620).

13. Procédé selon l'une des revendications précédentes, **caractérisé en ce que** la proportion source donnée (Psource) de NO gazeux est comprise entre 100 et 1000 ppm en volume, de préférence le reste du mélange gazeux est de l'azote.

FIG.1

# EP 2 581 103 A1

Europäisches
Patentamt

European
Patent Office

Office européen
des brevets

# RAPPORT DE RECHERCHE EUROPEENNE

Numéro de la demande

EP 12 18 4951

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X | EP 0 839 546 A2 (OHMEDA INC [US]) 6 mai 1998 (1998-05-06) | 1 | INV. A61M16/12 A62B27/00 |
| Y | * abrégé; figure 3 * <br> * colonne 1, ligne 1 - colonne 5, ligne 40 * | 2-13 | |
| Y | WO 2004/024053 A2 (ETHICON ENDO SURGERY INC [US]) 25 mars 2004 (2004-03-25) <br> * abrégé; figure 3 * <br> * alinéas [0004], [0512], [0014], [0015], [0025], [0032] - [0036] * | 2-13 | |
| Y | US 2005/056079 A1 (NAGY DONALD B [US] ET AL) 17 mars 2005 (2005-03-17) <br> * abrégé; figures 1-3 * <br> * alinéas [0001] - [0011], [0017] - [0031] * | 2-13 | |
| Y | JP 2008 249455 A (SHIMADZU CORP) 16 octobre 2008 (2008-10-16) <br> * le document en entier * | 8 | DOMAINES TECHNIQUES RECHERCHES (IPC) |
| Y | US 2005/273016 A1 (COLMAN LEWIS [IL] ET AL) 8 décembre 2005 (2005-12-08) <br> * le document en entier * | 2-13 | A61M A62B |

Le présent rapport a été établi pour toutes les revendications

| Lieu de la recherche | Date d'achèvement de la recherche | Examinateur |
|---|---|---|
| Munich | 11 janvier 2013 | Lalinde, Rafael |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

........................................................................

& : membre de la même famille, document correspondant

EPO FORM 1503 03.82 (P04C02)

**ANNEXE AU RAPPORT DE RECHERCHE EUROPEENNE
RELATIF A LA DEMANDE DE BREVET EUROPEEN NO.**

EP 12 18 4951

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

11-01-2013

| Document brevet cité au rapport de recherche | | Date de publication | Membre(s) de la famille de brevet(s) | | Date de publication |
|---|---|---|---|---|---|
| EP 0839546 | A2 | 06-05-1998 | CA | 2211748 A1 | 02-04-1998 |
| | | | EP | 0839546 A2 | 06-05-1998 |
| WO 2004024053 | A2 | 25-03-2004 | AU | 2003270685 A1 | 30-04-2004 |
| | | | EP | 1542755 A2 | 22-06-2005 |
| | | | US | 2004107965 A1 | 10-06-2004 |
| | | | WO | 2004024053 A2 | 25-03-2004 |
| US 2005056079 | A1 | 17-03-2005 | DE | 102004040455 A1 | 04-05-2005 |
| | | | US | 2005056079 A1 | 17-03-2005 |
| JP 2008249455 | A | 16-10-2008 | AUCUN | | |
| US 2005273016 | A1 | 08-12-2005 | AU | 5243100 A | 28-12-2000 |
| | | | EP | 1198198 A2 | 24-04-2002 |
| | | | JP | 2003524149 A | 12-08-2003 |
| | | | US | 6969357 B1 | 29-11-2005 |
| | | | US | 2005273016 A1 | 08-12-2005 |
| | | | WO | 0074553 A2 | 14-12-2000 |

EPO FORM P0460

Pour tout renseignement concernant cette annexe : voir Journal Officiel de l'Office européen des brevets, No.12/82

## RÉFÉRENCES CITÉES DANS LA DESCRIPTION

**Documents brevets cités dans la description**

- US 5558083 A
- EP 872254 A